(19) Europäisches Patentamt
European Patent Office
Office européen des brevets

(11) Publication number: 0 170 748 A1

# (12) EUROPEAN PATENT APPLICATION

(21) Application number: 84305395.0

(22) Date of filing: 08.08.84

(51) Int. Cl.⁴: **A 61 K 7/06**

(43) Date of publication of application: 12.02.86 Bulletin 86/7

(84) Designated Contracting States: AT BE CH DE FR GB IT LI LU NL SE

(71) Applicant: **NIHON NOHYAKU CO., LTD., 1-2-5, Nihonbashi, Chuo-ku Tokyo (JP)**

(72) Inventor: **Inishi, Shizuo, 361 Nakamichi, Hashimoto-shi (JP)**
Inventor: **Tanaka, Tetsuji, 47-8, Shiroyamadai-1-chome, Hashimoto-shi (JP)**
Inventor: **Tsuchiya, Katsuo, 11-19 Nankadai-5-chome, Kawachinagano-shi Osaka-fu (JP)**
Inventor: **Hayashi, Masahiro, 3-5 Miharadai-4-chome, Sakai-shi (JP)**

(74) Representative: **Grundy, Derek George Ritchie et al, CARPMAELS & RANSFORD 43, Bloomsbury Square, London WC1A 2RA (GB)**

## (54) Hair tonic composition.

(57) A hair tonic composition containing as an active ingredient a compound represented by the general formula (I),

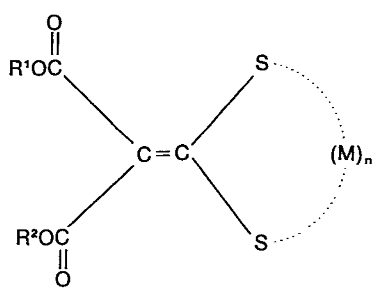

(I)

wherein $R^1$ and $R^2$ may be the same of different and each are a lower alkyl group, M is an inorganic or organic salt having a valency of 1 or 2, and n is 2 when M has a valency bond of 1, and n is 1 when M has a valency of 2.

EP 0 170 748 A1

# HAIR TONIC COMPOSITION

## FIELD OF THE INVENTION

The present invention relates to a hair tonic composition. More particularly, the present invention relates to a hair tonic composition characterized by containing as an active ingredient a compound represented by the following general formula:

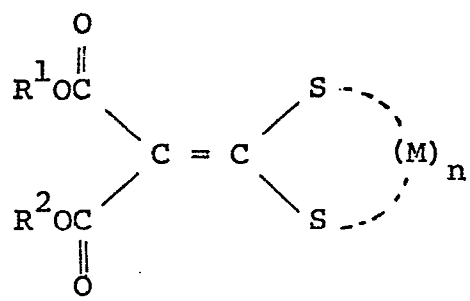

(I)

wherein $R^1$ and $R^2$ may be the same or different and each are a lower alkyl group, M is an inorganic or organic salt having a valency of 1 or 2, and _n_ is 2 when M has a valency of 1 and _n_ is 1 when M has a valency of 2.
In the description and claims 'lower' indicates a straight or branched chain alkyl group having from 1 to 6 carbon atom.

## DESCRIPTION OF THE PRIOR ART

Many hair tonics each containing various active ingredients have been known. They contain, for example, disinfectants, keratolytics, hormones, vitamins, amino acids, vasodilators, etc. and are used for purposes of prophylaxis or treatment of alopecia.

These hair tonics are claimed to be effective

for prophylaxis and treatment of dandruff, pruritus, excessive hair falling, etc. and to promote hair growth and development. However, there is no hair tonic available yet which has a fully satisfactory effect.

As a result of research on substances having hair tonic effects as well as hair-growing effects, the present inventors found that compounds represented by the general formula (I) have these effects without exhibiting toxicity. Based on this finding, the present invention has been accomplished.

SUMMARY OF THE INVENTION

An object of the present invention is to provide a hair tonic composition capable of preventing excessive hair falling of humans.

Another object of the present invention is to provide a hair tonic composition which is applied to those animals whose wools or furs are utilized by humans such as sheep, fox and mink and which promotes the growth of wools or furs of the animals.

DETAILED DESCRIPTION OF PREFERRED EMBODIMENTS

The compounds represented by the general formula (I) which are used as an active component of the hair tonic composition of the present invention are known in Japanese Patent Publication No. 44327/1974 as agricultural and horticultural fungicides effective for Piricularia oryzae BRI. et. CAV. and Hypochnus sasakii SHIRAI. Shown

in Table 1 are typical examples of the compounds represented by the general formula (I), however, the active ingredients used in the hair tonic composition of the present invention are not restricted to the compounds shown in Table 1.

General formula (I)

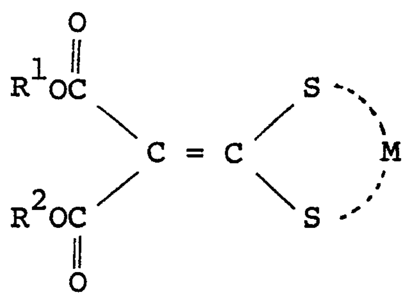

(I)

The active ingredients used in the hair tonic composition of the present invention are not restricted to the compounds shown in Table 1 and further include other salts, namely, (a) inorganic salts such as ammonia salts and the like, (b) organic salts, and (c) these salts having crystal water.

Table 1

| No. | $R^1$ | $R^2$ | M | Color and decomposing point |
|---|---|---|---|---|
| 1 | $i-C_3H_7$ | $i-C_3H_7$ | Na | Light yellow. Begins to decompose at 70°C and decomposes vigorously at 80°C. |
| 2 | $i-C_3H_7$ | $i-C_3H_7$ | K | Light yellow. Begins to decompose at 70°C and decomposes vigorously at 120°C. |
| 3 | $CH_3$ | $CH_3$ | Ca | Light yellow. Decomposes in white fume at 290°C or above. |
| 4 | $CH_3$ | $CH_3$ | Cu | Dark black. Melts at 146° to 148°C. |
| 5 | $CH_3$ | $CH_3$ | Mn | Light brown. Turns black and decomposes at 271°C or above. |
| 6 | $CH_3$ | $CH_3$ | Co | Liver brown. Carbonizes and decomposes at 245° to 251°C. |
| 7 | $C_2H_5$ | $C_2H_5$ | Ba | Light yellow. Decomposition point is higher than 300°C |
| 8 | $C_2H_5$ | $C_2H_5$ | Ca | Yellow. Decomposes at 285°C or above. |
| 9 | $C_2H_5$ | $C_2H_5$ | Fe | Light brown. Turns black at 250°C or above. |
| 10 | $C_2H_5$ | $C_2H_5$ | Ni | Black. Decomposes at 289°C or above. |
| 11 | $n-C_3H_7$ | $n-C_3H_7$ | Ca | Yellow. Turns brown and decomposes at 275°C or above. |
| 12 | $i-C_3H_7$ | $i-C_3H_7$ | Mn | Brown. Turns black at 263°C or above. |
| 13 | $i-C_3H_7$ | $i-C_3H_7$ | Fe | Light brown. Decomposes at 249°C or above. |
| 14 | $i-C_3H_7$ | $i-C_3H_7$ | Zn | Yellow. Turns brown and decomposes at 280°C. |
| 15 | $i-C_3H_7$ | $i-C_3H_7$ | Cu | Black. Decomposes at 122° to 124°C. |
| 16 | $n-C_4H_9$ | $n-C_4H_9$ | Ba | Light yellow. Decomposition point is higher than 300°C. |

| | | | | |
|---|---|---|---|---|
| 17 | i-$C_4H_9$ | i-$C_4H_9$ | Fe | Liver brown. Turns black and decomposes at 251°C or above. |
| 18 | i-$C_4H_9$ | i-$C_4H_9$ | Cu | Blackish blue. Melts and sublimates at 161° to 165°C. |
| 19 | t-$C_4H_9$ | t-$C_4H_9$ | Ca | Yellow. Turns grayish white at 300°C. |

The hair tonic composition of the present invention can be prepared in general forms of hair care products such as hair tonic, hair lotion, hair cream, emulsion, shampoo, rinse and the like, according to ordinary methods.

As to the base materials for the hair tonic composition of the present invention, there are used, for example, alcohols, oils and fats, surfactants and the like. Other active ingredients such as resorcinol, hormones, vitamins, amino acids, menthol and other perfumes may also be used in the hair tonic composition of the present invention.

The amount of active ingredient used in the hair tonic composition of the present invention may be optionally determined so as to meet various usage requirements, and can generally be 0.01 to 10% by weight based on the total amount of hair tonic composition.

The hair tonic composition of the present invention can be applied to the hair portion 1 to 3 times a day.

Examples of test and formulations of the hair

tonic composition of the present invention are illustrated below. However, the hair tonic composition of the present invention is not restricted only to these tests and formulations.

TEST 1

Hairs of 6 Hartley strain guinea pigs (male and body weight of about 300 g) were cut at their backs by the use of a hair clipper so as not to injure their skins. To a portion of a 3 cm x 3 cm square which was a part of the hair-removed back of each guinea pig and at one side of the two divided by the spine, there was coated 0.3 ml of an ethanol solution containing 1% of the compound No. 14 mentioned in Table 1, once a day for 7 consecutive days. To another portion of a 3 cm x 3 cm square which was at the other side of the two divided by the spine of each guinea pig, there was coated 0.3 ml of an ethanol solution not containing the compound No. 14, in the same frequency and for the same period. After completion of the coating, each 20 haris were depilated from the above two portions and their lengths were measured. These lengths minus the lengths at the start of the test were lengths elongated during the test period. The results are shown in Table 2.

Table 2

| Guinea pig No. | Length of hair elongated (Average of 20 hairs) (mm) | | Average difference (mm) |
|---|---|---|---|
| | Hair tonic composition of the present invention | Control | |
| 1 | 5.315 | 5.111 | 0.204 |
| 2 | 5.275 | 4.894 | 0.381 |
| 3 | 4.670 | 4.475 | 0.195 |
| 4 | 5.140 | 4.905 | 0.235 |
| 5 | 4.443 | 4.143 | 0.300 |
| 6 | 4.372 | 4.170 | 0.202 |

Shown next is a test wherein hair tonic compositions of the present invention were applied to moustaches of 12 healthy human males.

TEST 2

To an ointment base prepared by mixing a hydrophilic ointment and a white petrolatum at a 1:1 ratio was added 0.1% of the previously mentioned compound No. 1 or 14, whereby two kinds of creams were prepared. An appropriate amount of either of these creams was rubbed into the right or left moustache portion of each examinee

2 to 3 times a day and, after each application, the cream-applied moustache portion was thoroughly massaged. The same amount of the above ointment base *per se* was rubbed into another moustache portion at the same frequency per day in the same manner. This treatment was continued for 10 consecutive days. Hairs of respective moustache portions were cut off with a safety razor. The lengths of 20 hairs of each portion were measured, and their averages as well as the difference between the two averages were calculated. The results are shown in Table 3.

Table 3

| Compound No. | Examinee (Male) | | Hair length (Average of 20 hairs) (mm) | | Difference (mm) |
|---|---|---|---|---|---|
| | No. | Age | Hair tonic composition of the present invention | Control | |
| 1 | 1 | 30 | 3.495 | 3.310 | 0.185 |
| 1 | 2 | 30 | 3.535 | 3.515 | 0.020 |
| 1 | 3 | 30 | 3.428 | 3.212 | 0.216 |
| 1 | 4 | 40 | 3.663 | 3.354 | 0.310 |
| 14 | 5 | 40 | 3.935 | 3.530 | 0.405 |
| 14 | 6 | 40 | 3.640 | 3.100 | 0.540 |
| 14 | 7 | 33 | 3.402 | 3.395 | 0.007 |
| 14 | 8 | 33 | 3.565 | 3.190 | 0.375 |
| 14 | 9 | 37 | 3.495 | 3.145 | 0.350 |

| | | | | | |
|---|---|---|---|---|---|
| 14 | 10 | 30 | 3.475 | 3.460 | 0.015 |
| 14 | 11 | 31 | 3.250 | 2.915 | 0.335 |
| 14 | 12 | 38 | 3.210 | 2.730 | 0.480 |

In the above Test 2, a significant difference between the use of the hair tonic composition of the present invention and the non-use of the same composition was clearly observed in 9 examinees of total 12.

TEST 3

The previously mentioned compound No. 14 was mixed into corn starch at a proportion of 10%. Each 10 mg of the resulting powder was encapsulated, whereby capsules containing the compound No. 14 were prepared.

At the time of use of these capsules, the powder in capsule was taken out onto a plam and dissolved in 1 ml of a diluent consisting of water-ethanol-propylene glycol (5:4.5:0.5) and a perfume on the palm.

The resulting solution was thoroughly coated on the head of each examinee by both hands.

This treatment was conducted 2 times a day for several consequent weeks. The results are shown in Table 4.

Table 4

| Examinee | | Period of coating (day) | Hair tonic effect |
|---|---|---|---|
| No. | Age | | |
| 1 | 34 | 24 | The number of falling hairs decreased. |
| 2 | 31 | 19 | The number of falling hairs decreased. |
| 3 | 31 | 41 | The gloss of hairs improved. |
| 4 | 52 | 40 | The number of falling hairs decreased. |

In the following test, the toxicity of the compound No. 14 was examined.

TEST 4

(1) The compound No. 14 dissolved in olive oil was orally administered to 10 SD rats (male, 6 week old) at a ratio of 5,000 mg per kg of body weight. Thereafter, the conditions of these rats as well as their mortality observed for 14 days. As a result, there was neither toxication nor mortality. Therefore, the $LD_{50}$ value of the compound No. 14 is larger than 5,000 mg/kg.

(2) Separately, hairs were depilated from Japanese white rabbits at their backs by the use of a depilatory cream. Then, the depilated portion of each rabbits was coated with 1 or 5 mg of the compound No. 14 for 24 hours. Thereafter, the skin condition of the compound No. 14-coated portion was observed for 7 days. There was no change

of skin at any of the compound No. 14-coated portions. Accordingly, it was proved that the compound No. 14 shows substantially no primary stimulation to the skin.

Next, examples of recipe of the hair tonic composition of the present invention are shown, however, the hair tonic composition of the present invention is not restricted only to these examples.

EXAMPLE 1

| | |
|---|---|
| Compound No. 1 | 0.5 g |
| Salicylic acid | 1.0 g |
| Resorcinol | 2.0 g |
| Glycerine | 2.0 g |
| Phenol | 1.0 g |
| Castor oil | 1.0 g |
| Lavender oil | 10.0 ml |

The above materials are dissolved in ethanol to make a lotion of a total volume of 100 ml.

EXAMPLE 2

| | |
|---|---|
| Compound No. 2 | 1.0 g |
| Peppermint oil | 0.6 g |
| Glycerine | 15.0 ml |
| Lavender oil | 10.0 ml |

The above materials are dissolved in ethanol to make a lotion of a total volume of 100 ml.

EXAMPLE 3

| | |
|---|---|
| Compound No. 14 | 0.1 g |
| Calamine | 8.0 g |
| Sodium alginate | 1.25 g |
| Glycerine | 4.0 g |
| Methyl p-hydroxybenzoate | 0.2 g |
| Zinc oxide | 8.0 g |
| Tween 20 (tradename, for a polyoxyethylene sorbitan monolaurate) | 0.01 g |

The above materials are dissolved in purified water to make a lotion of a total volume of 100 ml.

EXAMPLE 4

| | |
|---|---|
| Compound No. 1 | 0.5 g |
| Potassium soap | 8.0 g |
| Peppermint oil | 0.6 g |
| Lavender oil | 10.0 ml |

The above materials are dissolved in ethanol to make a lotion of a total volume of 100 ml.

EXAMPLE 5

| | |
|---|---|
| Compound No. 14 | 0.5 g |
| Ethyl p-hydroxybenzoate | 0.025 g |
| Propyl p-hydroxybenzoate | 0.015 g |
| Sodium laurylsulfate | 1.5 g |
| Propylene glycol | 12.0 g |
| Stearyl alcohol | 22.0 g |

| | |
|---|---|
| White petrolatum | 25.0 g |
| Purified water | 38.96 g |

The above materials are melted and mixed to make an ointment of a total weight of 100 g.

EXAMPLE 6

| | |
|---|---|
| Compound No. 8 | 0.5 g |
| Polyethylene glycol 400 | 57.5 g |
| Polyethylene glycol 1500 | 20.0 g |
| Polyethylene glycol 4000 | 22.0 g |

The above materials are melted and mixed to make an ointment of a total weight of 100 g.

EXAMPLE 7

| | |
|---|---|
| Compound No. 1 | 0.1 g |
| Purified lanolin | 5.0 g |
| Bleached bees wax | 5.0 g |
| White petrolatum | 89.5 g |

The above materials are melted and mixed to make an ointment of a total weight of 100 g.

EXAMPLE 8

| | |
|---|---|
| Compound No. 14 | 0.5 g |
| Glycerine monostearate | 18.0 g |
| Spermaceti | 5.0 g |
| Diethylene glycol monoethyl ether | 5.0 g |

| | |
|---|---|
| Peppermint oil | 1.1 g |
| Purified water | q.s. |

The above materials are melted and mixed to make an ointment of a total weight of 100 g.

EXAMPLE 9

| | |
|---|---|
| Compound No. 5 | 0.5 g |
| High viscosity type carboxymethyl | |
| Cellulose | 2.0 g |
| Glycerine | 80.0 g |
| Refined water | 17.5 g |

The above materials are melted and mixed to make an ointment of a total weight of 100 g.

WHAT IS CLAIMED IS:

1. A hair tonic composition characterized by containing as an active ingredient a compound represented by the general formula (I)

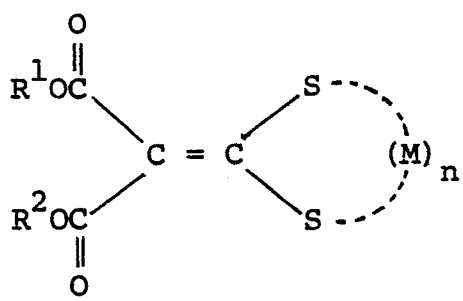

(I)

wherein $R^1$ and $R^2$ may be the same or different and each are a lower alkyl group, M is an inorganic or organic salt having a valency of 1 or 2, and n is 2 when M has a valency of 1 and n is 1 when M has a valency of 2.

2. A hair tonic composition according to Claim 1, which contains as an active ingredient a compound represented by the general formula (I) wherein $R^1$ and $R^2$ each are a lower alkyl group selected from the group consisting of $CH_3$, $C_2H_5$, i-$C_3H_7$ and i-$C_4H_9$; M is a metal atom of a valency of 1 or 2 selected from the group consisting of K, Na, Ca, Ba, Cu, Mn, Co, Ni and Fe, and n is 2 when M is a metal atom of a valency of 1 and n is 1 when M is a metal atom of a valency of 2.

3. A hair tonic composition according to Claim 2, which contains as an active ingredient a compound represented by the general formula (I) wherein $R^1$ and $R^2$ each are i-$C_3H_7$, M is Na and n is 2.

4. A hair tonic composition according to Claim 2,

which contains as an active ingredient a compound represented by the general formula (I) wherein $R^1$ and $R^2$ each are $i\text{-}C_3H_7$, M is K and n is 2.

5. A hair tonic composition according to Claim 2, which contains as an active ingredient a compound represented by the general formula (I) wherein $R^1$ and $R^2$ each are $i\text{-}C_3H_7$, M is Zn and n is 1.

6. A hair tonic composition according to Claim 2, which contains as an active ingredient a compound represented by the general formula (I) wherein $R^1$ and $R^2$ each are $CH_3$, M is Mn and n is 1.

7. A hair tonic composition according to Claim 2, which contains as an active ingredient a compound represented by the general formula (I) wherein $R^1$ and $R^2$ each are $C_2H_5$, M is Ca and n is 1.

8. A hair tonic composition according to any of Claims 1 to 7, wherein the amount of active ingredient is 0.01 to 10% by weight based on the total amount of hair tonic composition.

European Patent Office

# EUROPEAN SEARCH REPORT

0170748

Application number

EP 84 30 5395

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | CHEMICAL ABSTRACTS, vol. 83, no. 9, 1st September 1975, page 223, no. 73457c, Columbus, Ohio, US; & JP - A - 74 55827 (NIHON NOHYAKU CO., LTD.) 30-05-1974 | | A 61 K 7/06 |
| A | CHEMICAL ABSTRACTS, vol. 84, no. 5, 2nd February 1976, page 132, no. 26893c, Columbus, Ohio, US; & JP - A - 75 116 636 (NIHON NOHYAKU CO., LTD.) 12-09-1975 | | |
| A | CHEMICAL ABSTRACTS, vol. 100, no. 15, 9th April 1984, page 61, no. 115000a, Columbus, Ohio, US; & JP - A - 58 222 019 (DAIICHI KOGYO SEIYAKU CO., LTD. NIHON NOHYAU CO., LTD.) 23-12-1983 | | TECHNICAL FIELDS SEARCHED (Int. Cl.4) |
| A | GB-A-2 025 415 (L'OREAL) * Claim 1 * | | A 61 K<br>C 07 C |
| A | DE-A-2 630 947 (KAO SOAP) * Page 8, lines 19-24; claim 1 * | | |

The present search report has been drawn up for all claims

| Place of search | Date of completion of the search | Examiner |
|---|---|---|
| THE HAGUE | 17-04-1985 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82